(19) Europäisches Patentamt / European Patent Office / Office européen des brevets

(11) **EP 3 723 003 B1**

(12) **FASCICULE DE BREVET EUROPEEN**

(45) Date de publication et mention
de la délivrance du brevet:
**07.06.2023 Bulletin 2023/23**

(51) Classification Internationale des Brevets (IPC):
**G06K 19/073** (2006.01)    **A61B 5/053** (2021.01)
**G06K 19/00** (2006.01)

(21) Numéro de dépôt: **20168320.8**

(22) Date de dépôt: **06.04.2020**

(52) Classification Coopérative des Brevets (CPC):
**G06K 19/07354; A61B 5/053; A61B 5/1172;**
**A61B 5/6826; G06V 40/12;** A61B 5/6898;
A61B 2503/12; G06K 19/0718

(54) **CARTE À PUCE COMPORTANT UN CAPTEUR D'EMPREINTES DIGITALES**

CHIPKARTE MIT INTEGRIERTEM FINGERABDRUCKSENSOR

A SMARTCARD INCLUDING A FINGERPRINT SENSOR

(84) Etats contractants désignés:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priorité: **12.04.2019 FR 1903921**

(43) Date de publication de la demande:
**14.10.2020 Bulletin 2020/42**

(73) Titulaire: **Idemia Identity & Security France**
**92400 Courbevoie (FR)**

(72) Inventeurs:
• **RAGOT, Marcelin**
**92400 COURBEVOIE (FR)**
• **BRICOUT, Franck**
**92400 COURBEVOIE (FR)**
• **THIEBOT, Alain**
**92400 COURBEVOIE (FR)**

(74) Mandataire: **Idemia**
**2, place Samuel de Champlain**
**92400 Courbevoie (FR)**

(56) Documents cités:
**EP-A1- 0 983 572       EP-B1- 0 983 572**
**WO-A2-2006/014205     US-B1- 6 343 140**
**US-B2- 10 223 569**

**EP 3 723 003 B1**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

## Description

L'invention concerne le domaine des cartes à puce comportant un capteur d'empreintes digitales.

### ARRIERE PLAN DE L'INVENTION

Certaines cartes à puce récentes, et notamment des cartes de paiement, sont équipées d'un capteur d'empreintes digitales (qui est par exemple un capteur optique ou capacitif).

Grâce au capteur d'empreintes digitales, il n'est plus nécessaire de taper un code personnel à quatre chiffres pour authentifier l'utilisateur d'une carte à puce : il suffit à l'utilisateur de poser un doigt sur le capteur d'empreintes digitales.

Des individus malintentionnés tentent de contourner frauduleusement ce type de dispositif d'authentification en utilisant un faux doigt. Il est en effet possible que certains individus cherchent à reproduire une empreinte digitale.

Le document WO 2006/014205 A2 divulgue une carte à puce de l'art antérieur, qui est équipée d'un capteur d'empreintes digitales.

### OBJET DE L'INVENTION

L'invention a pour objet de sécuriser l'authentification, réalisée par un capteur d'empreintes digitales d'une carte à puce, d'un utilisateur de ladite carte à puce.

### RESUME DE L'INVENTION

En vue de la réalisation de ce but, on propose une carte à puce comportant un capteur d'empreintes digitales, caractérisée en ce que la carte à puce comporte en outre un circuit de détection comprenant au moins une première électrode et une deuxième électrode isolées entre elles et formant un circuit ouvert dans le circuit de détection, la première électrode et la deuxième électrode étant disposées pour que, lorsqu'un utilisateur applique un premier doigt sur le capteur d'empreintes digitales, le premier doigt soit en contact avec la première électrode et l'utilisateur puisse appliquer un deuxième doigt sur la deuxième électrode de manière à former une boucle refermant le circuit ouvert, le circuit de détection comprenant de plus un circuit de filtrage relié à la première électrode et à la deuxième électrode, un générateur agencé pour générer des premiers signaux aux bornes du circuit de filtrage, et des moyens de traitement agencés pour acquérir des deuxièmes signaux sur l'une des première électrode et deuxième électrode et pour détecter à partir des deuxièmes signaux si le premier doigt et le deuxième doigt comprennent un doigt factice.

La boucle, qui s'étend entre l'extrémité du premier doigt, par exemple le pouce d'une main de l'individu, et l'extrémité du deuxième doigt, par exemple l'index de la même main, forme un filtre dont l'impédance est très différente selon que les deux doigts sont des doigts humains ou bien comprennent un doigt factice. L'impédance de ce filtre influe sur le circuit de filtrage et donc sur les deuxièmes signaux dont l'analyse permet de détecter si un doigt factice est utilisé ou non.

On propose de plus une carte à puce telle que celle qui vient d'être décrite, dans laquelle la première électrode est un contour du capteur d'empreintes digitales.

On propose en outre une carte à puce telle que celle qui vient d'être décrite, dans laquelle la première électrode est formée sur une surface d'acquisition du capteur d'empreintes digitales.

On propose de plus une carte à puce telle que celle qui vient d'être décrite, dans laquelle la première électrode et la deuxième électrode sont positionnées sur deux faces opposées de la carte à puce.

On propose par ailleurs une carte à puce telle que celle qui vient d'être décrite, dans laquelle les premiers signaux comprennent des signaux sinusoïdaux ayant chacun une fréquence d'émission distincte, les moyens de traitement étant agencés pour acquérir un deuxième signal pour chaque fréquence d'émission.

On propose de plus une carte à puce telle que celle qui vient d'être décrite, dans laquelle les moyens de traitement sont agencés pour déterminer une tension représentative de chaque deuxième signal, de manière à obtenir un signal de tension en fonction de la fréquence d'émission.

On propose en outre une carte à puce telle que celle qui vient d'être décrite, dans laquelle les moyens de traitement sont agencés pour dériver le signal de tension en fonction de la fréquence d'émission pour obtenir un signal dérivé, et pour comparer le signal dérivé avec des premières données de référence stockées dans une mémoire de la carte à puce.

On propose de plus une carte à puce telle que celle qui vient d'être décrite, dans laquelle les premières données de référence proviennent de courbes de référence obtenues, préalablement ou pendant la fabrication de la carte à puce, par des mesures réalisées sur des doigts humains réels.

On propose de plus une carte à puce telle que celle qui vient d'être décrite, dans laquelle les premières données de référence comprennent des coefficients de fonctions d'interpolation des courbes de référence.

On propose de plus une carte à puce telle que celle qui vient d'être décrite, dans laquelle les premières données de référence comprennent des points remarquables des courbes de référence.

On propose de plus une carte à puce telle que celle qui vient d'être décrite, dans laquelle les moyens de traitement sont agencés pour estimer un déphasage de chaque deuxième signal, pour produire un signal de déphasage en fonction de la fréquence d'émission, et pour comparer le signal de déphasage avec des deuxièmes données de référence stockées dans une mémoire de la carte à puce.

On propose de plus une carte à puce telle que celle qui vient d'être décrite, dans laquelle les premiers signaux sont des signaux monofréquences.

L'invention sera mieux comprise à la lumière de la description qui suit d'un mode de mise en oeuvre particulier non limitatif de l'invention.

BREVE DESCRIPTION DES DESSINS

Il sera fait référence aux dessins annexés, parmi lesquels :

[Fig.1] la figure 1 représente une carte à puce selon l'invention ;
[Fig.2] la figure 2 représente un circuit de détection de la carte à puce selon l'invention ;
[Fig.3] la figure 3 est un graphique comprenant une courbe d'un signal dérivé de référence en fonction de la fréquence d'émission des premiers signaux ;
[Fig.4] la figure 4 est un graphique comprenant une courbe de référence minimale, une courbe de référence moyenne et une courbe de référence maximale ;
[Fig.5] la figure 5 est un graphique comprenant les courbes des fonctions d'interpolation des courbes de référence de la figure 4 ;
[Fig.6] la figure 6 est un graphique comprenant une courbe d'un signal de déphasage de référence en fonction de la fréquence d'émission des premiers signaux.

DESCRIPTION DETAILLEE DE L'INVENTION

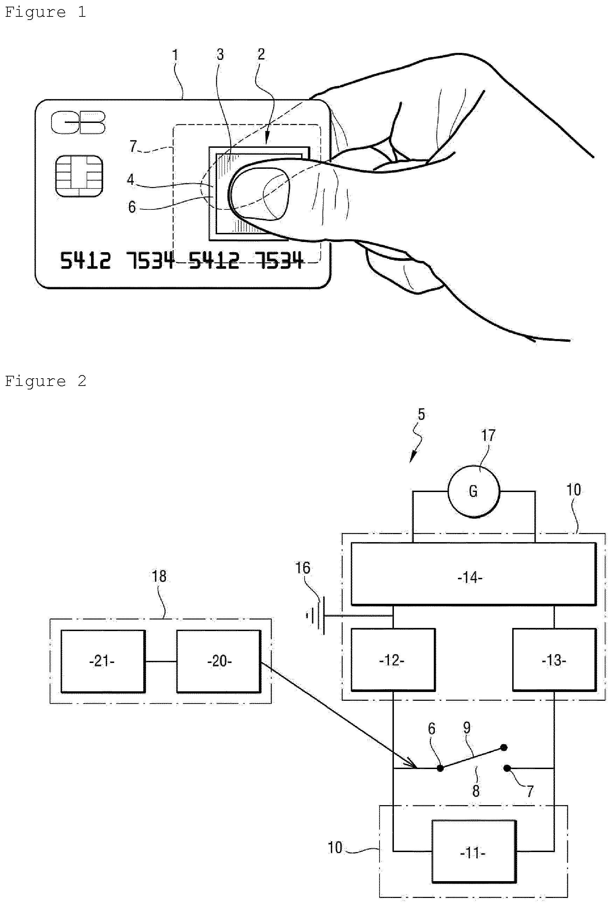

En référence à la figure 1, une carte à puce selon l'invention 1 comporte tout d'abord un capteur d'empreintes digitales 2.

Le capteur d'empreintes digitales 2 comprend une surface d'acquisition 3 encadrée par un contour 4 (ou *bezel,* en anglais). Le contour 4 est fabriqué en un matériau métallique. Pour s'authentifier, un utilisateur de la carte à puce 1 doit poser le pouce d'une de ses mains sur la surface d'acquisition 3 du capteur d'empreintes digitales 2. Le capteur d'empreintes digitales 2 produit alors un signal pour valider ou non l'authentification de l'individu.

La carte à puce 1 comporte de plus un circuit de détection.

En référence à la figure 2, le circuit de détection 5 comprend tout d'abord une première surface électriquement conductrice formant une première électrode 6 et une deuxième surface électriquement conductrice formant une deuxième électrode 7.

La première électrode 6 est intégrée dans le capteur d'empreintes digitales 2, et est formée en l'occurrence par le contour 4 de la surface d'acquisition 3.

Alternativement, la première électrode 6 pourrait être formée directement sur la surface d'acquisition 3. La première électrode 6 peut ainsi être une grille métallique qui s'étend sur la surface d'acquisition 3.

La deuxième électrode 7 est formée sur une face de la carte à puce 1 opposée à celle sur laquelle est positionnée la première électrode 6. La deuxième électrode 7 est fabriquée en un matériau électriquement conducteur, par exemple en métal ou bien en un plastique laminé dans lequel est intégrée une poudre de graphite.

La première électrode 6 et la deuxième 7 électrode sont isolées entre elles et forment un circuit ouvert 8 dans le circuit de détection 5.

La première électrode 6 et la deuxième électrode 7 sont disposées de sorte que, lorsque l'utilisateur applique son pouce sur la surface d'acquisition 3 du capteur d'empreintes digitales 2 et utilise l'index de la même main pour saisir la carte à puce 1, le pouce est en contact avec la première électrode 6 et l'index est en contact avec la deuxième électrode 7 de manière à former une boucle refermant le circuit ouvert 8 dans le circuit de détection 5. La première électrode 6 et la deuxième électrode 7 forment ainsi un interrupteur 9 normalement ouvert, et refermé lorsque deux doigts (humains ou factices) sont appliqués sur la première électrode 6 et sur la deuxième électrode 7.

Le circuit de détection 5 comporte de plus un circuit de filtrage 10 relié à la première électrode 6 et à la deuxième électrode 7. Le circuit de filtrage 10 forme un filtre passe-bas, mais pourrait former un autre type de filtre, par exemple un filtre passe-haut ou un filtre passe-bande. Le circuit de filtrage 10 comprend ici un premier filtre 11, un deuxième filtre 12, un troisième filtre 13 et un étage de pré-filtrage 14. Les premier, deuxième et troisième filtres 11, 12, 13 et l'étage de pré-filtrage 14 comprennent chacun une capacité et/ou une résistance et/ou une inductance. Le premier filtre 11 est monté entre la première électrode 6 et la deuxième électrode 7. Le deuxième filtre 12 est monté entre une masse électrique 16 et la première électrode 6. Le troisième filtre 13 est relié à la deuxième électrode 7. L'étage de pré-filtrage 14 est monté entre le deuxième filtre 12 et le troisième filtre 13.

Le circuit de détection 5 comporte de plus un générateur 17 qui produit et applique des premiers signaux aux bornes du circuit de filtrage 10, en l'occurrence entre des bornes de l'étage de pré-filtrage 14. L'étage de pré-filtrage 14 est optionnel et permet d'éviter de générer des signaux aberrants.

Le circuit de détection 5 comporte en outre des moyens de traitement 18 qui acquièrent et analysent des deuxièmes signaux sur la première électrode 6 et/ou sur la deuxième électrode 7, en l'occurrence sur la première électrode 6.

Les moyens de traitement 18 comprennent un convertisseur analogique numérique 20 ayant une entrée reliée à la première électrode 6. Le convertisseur analogique numérique 20 acquiert et numérise les deuxièmes signaux.

Les moyens de traitement 18 comprennent aussi un processeur 21 ayant une entrée reliée à une sortie du convertisseur analogique numérique 20. Un logiciel, programmé dans le processeur 21, analyse les deuxièmes signaux.

Le processeur 21 est avantageusement un processeur sécurisé. On note qu'à la place du processeur 21, il serait possible d'utiliser un microcontrôleur, un FPGA, un ASIC, etc.

Le générateur 17 génère de manière continue les premiers signaux. Chaque premier signal est un signal sinusoïdal ayant une fréquence d'émission distincte. Les fréquences des premiers signaux varient ici entre 1kHz et 70kHz. La valeur crête de chaque premier signal est constante et est comprise entre 1,1 et 5V, sous un courant de 5mA, ce qui correspond aux valeurs classiquement utilisées par un processeur 21.

Chaque deuxième signal résulte d'un premier signal distinct et est aussi un signal sinusoïdal ayant pour fréquence la fréquence d'émission dudit premier signal.

L'amplitude de chaque deuxième signal dépend de l'influence sur le premier signal associé du circuit de filtrage 10 et de la boucle formée par les doigts (humain ou factices) qui referment le circuit ouvert 8.

Lorsque le pouce et l'index réels de l'utilisateur sont appliqués respectivement sur la première électrode 6 et sur la deuxième électrode 7, ils forment un filtre biologique, ou, plus précisément, une série complexe de filtres biologiques. Ici, on considère que le filtre biologique est principalement résistif et capacitif. On pourrait aussi considérer que le filtre biologique est un filtre inconnu, d'ordre et de valeurs inconnus. L'impédance de ce filtre biologique dépend des caractéristiques de la peau, des os, des liquides présents entre l'extrémité du pouce et de l'index de l'utilisateur.

L'impédance de ce filtre biologique est variable selon les individus mais est propre au corps humain. On note que l'os scaphoïde, situé entre le pouce et l'index, est un os volumineux et peu irrigué, de sorte que la réponse qu'il engendre est très significative.

Ainsi, lorsqu'un faux doigt est utilisé, une boucle est formée et referme bien le circuit ouvert 8, mais cette boucle forme un filtre aux caractéristiques différentes, qui influe différemment sur le circuit de filtrage 10 et donc sur les deuxièmes signaux. Comme le premier filtre 11, le deuxième filtre 12, le troisième filtre 13 et l'étage de pré-filtrage 14 ont des caractéristiques connues, les moyens de traitement 18 peuvent analyser les deuxièmes signaux pour déterminer si le pouce et l'index comprennent ou non un doigt factice.

L'analyse des deuxièmes signaux consiste à réaliser des comparaisons avec des données de référence stockées dans une mémoire de la carte à puce 1.

Les données de référence sont obtenues à partir de mesures effectuées au cours d'une opération de calibration réalisée en usine ou en laboratoire, et donc préalablement ou au cours de la fabrication de la carte à puce 1. Les données de référence sont générées à partir d'une carte à puce de référence, et sont ensuite chargées dans la mémoire de nombreuses cartes à puce telles que la carte à puce 1. La carte à puce de référence n'est pas nécessairement une carte à puce « réelle », mais peut être un équipement intégré dans un banc de test et dimensionné pour simuler le comportement d'une carte à puce réelle.

Les données de référence comprennent des premières données de référence et des deuxièmes données de référence, qui sont obtenues de la manière suivante.

Les premiers signaux sont appliqués aux bornes du circuit de filtrage de la carte à puce de référence, alors que celle-ci est saisie par des doigts humains réels. Les deuxièmes signaux sont acquis par les moyens de traitement. Pour chaque deuxième signal, on produit une tension de référence représentative dudit deuxième signal. La tension de référence est par exemple une valeur crête du deuxième signal. On produit alors un signal de tension de référence en fonction de la fréquence d'émission. Pour chaque fréquence d'émission, la valeur du signal de tension de référence est égale à la tension de référence représentative du deuxième signal obtenu à partir du premier signal généré à ladite fréquence d'émission.

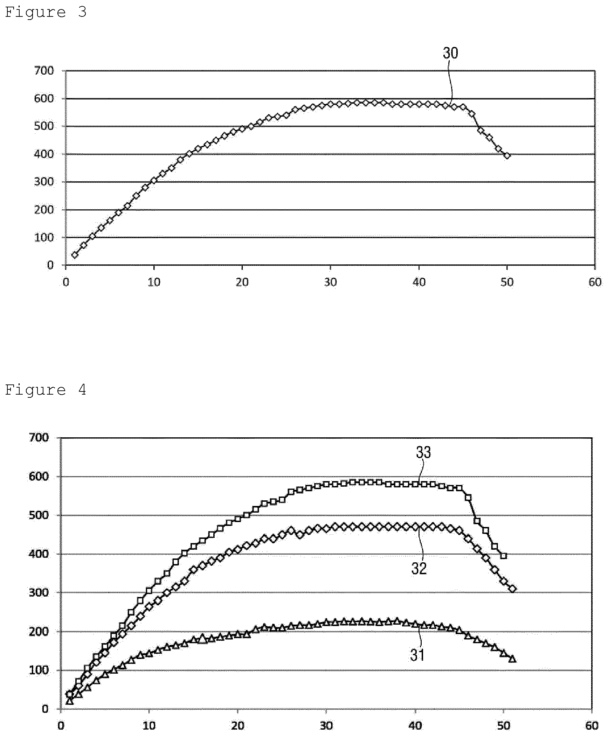

En référence à la figure 3, on dérive alors en fonction de la fréquence d'émission le signal de tension de référence pour obtenir un signal dérivé de référence dont la courbe est semblable à la courbe 30. On note ici que, sur la figure 3 comme sur les figures qui suivent, l'échelle utilisée pour la tension, en ordonnée, est une échelle linéaire arbitraire. En effet, la tension dépend de la tension des premiers signaux, qui peut varier entre 1,1V et 5V. Les fréquences, en abscisse, sont exprimées en kHz.

L'intérêt de la dérivation réside dans le fait que le corps humain a des caractéristiques électriques, et notamment une impédance biologique, qui varient entre les individus et, pour un même individu, qui peuvent varier dans le temps. Les mesures des caractéristiques électriques elles-mêmes dépendent donc de chaque individu particulier et sont relativement complexes à exploiter. Par contre, les valeurs dérivées des mesures en fonction de la fréquence, elles, sont relativement stables et caractéristiques du corps humain.

En référence à la figure 4, l'opération de calibration est réalisée sur une pluralité de doigts humains pour obtenir plusieurs courbes de signaux dérivés de référence, comprenant une courbe de référence minimale 31, une courbe de référence moyenne 32 et une courbe de référence maximale 33, qui couvrent un ensemble important des valeurs pouvant être obtenues par des doigts humains réels.

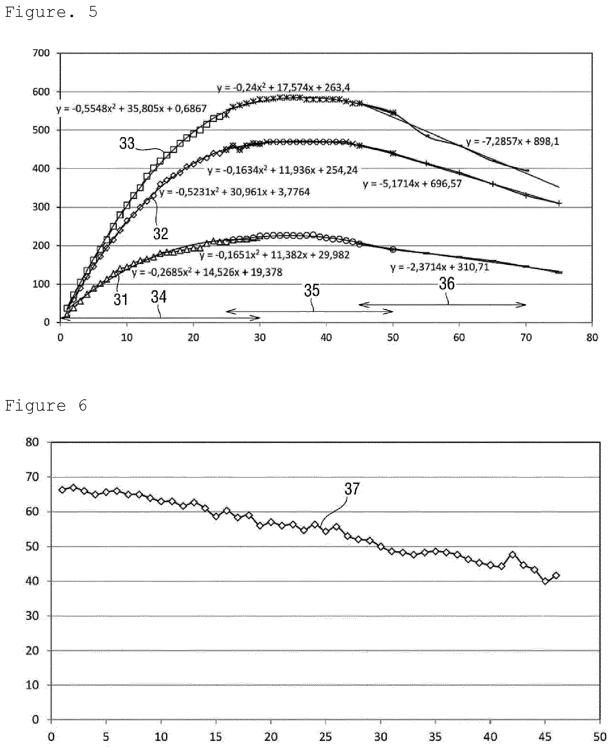

En référence à la figure 5, chaque courbe de référence est alors analysée selon un premier segment 34 compris entre 1kHz et 30kHz, un deuxième segment 35 compris entre 25kHz et 50kHz et un troisième segment 36 compris entre 45kHz et 70kHz.

On détermine alors une fonction d'interpolation pour chaque segment de chaque courbe.

La fonction d'interpolation pour le premier segment 34 est une première fonction polynomiale de second degré de la forme :

$$y = a.x^2 + b.x + c.$$

La fonction d'interpolation pour le deuxième segment 35 est une deuxième fonction polynomiale de second degré.

La fonction d'interpolation pour le troisième segment 36 est une fonction linéaire de la forme :

$$y = a.x + b.$$

Les premières données de référence sont les coefficients des fonctions d'interpolation des trois segments des trois courbes de référence 31, 32, 33, c'est-à-dire les coefficients a, b, c pour le premier segment 34 et le deuxième segment 35, et les coefficients a, b pour le troisième segment. Chaque coefficient peut avoir une valeur normalisée et être stocké sous la forme d'une donnée de 8 bits.

On note que la détermination des coefficients des fonctions d'interpolation nécessite une puissance de calcul relativement importante. Ces calculs sont réalisés en usine ou en laboratoire grâce à des machines disposant d'une puissance de calcul importante, par exemple grâce à des ordinateurs.

Alternativement, les premières données de référence pourraient être les points remarquables des courbes de référence (et non les coefficients des fonctions d'interpolation).

Il est dans ce cas avantageux de stocker au moins un point tous les 5kHz et, pour améliorer la précision de la détection, au moins un point tous les 1kHz.

La détermination des points remarquables nécessite une puissance de calcul moins élevée, mais un espace de stockage en mémoire plus important. De plus, le simple stockage des points remarquables ne permet pas d'obtenir des données de déphasage ou d'autres caractéristiques des courbes des signaux de référence. Cependant, ces caractéristiques pourraient être produites et stockées par ailleurs, de sorte que les premières données de référence comprennent à la fois les coefficients des fonctions d'interpolation et les points remarquables.

Les deuxièmes données de référence sont elles aussi obtenues au cours de mesures réalisées lors d'une opération de calibration sur des doigts humains réels.

On mesure le déphasage de chaque deuxième signal par rapport au premier signal associé. L'estimation du déphasage entre un premier signal et un deuxième signal est réalisée en utilisant un *trigger* et un chronomètre pour mesurer le temps entre un front du premier signal et un front de même sens du deuxième signal.

En référence à la figure 6, on produit un signal de déphasage de référence 37 en fonction de la fréquence d'émission des premiers signaux. Pour chaque fréquence d'émission, la valeur du signal de déphasage de référence est égale au déphasage du deuxième signal obtenu à partir du premier signal généré à ladite fréquence d'émission.

L'utilisation du déphasage est très intéressante, car le corps humain présente des caractéristiques de déphasage qui sont à la fois fortement distinctives et très stables.

Une fois que la carte à puce 1 est mise en service, chaque authentification de l'utilisateur, par exemple pour chaque paiement réalisé avec la carte à puce 1 si celle-ci est une carte de paiement, se passe de la manière suivante.

Le générateur 17 produit les premiers signaux de manière continue et les applique aux bornes du circuit de filtrage 10. Ici, par exemple, la fréquence d'émission varie par pas de 5kHz entre 1kHz et 70kHz. Les moyens de traitement 18 acquièrent les deuxièmes signaux et déterminent, pour chaque deuxième signal, la tension représentative dudit deuxième signal (c'est-à-dire à nouveau, par exemple, la tension crête). Les moyens de traitement 18 produisent un signal de tension en fonction de la fréquence d'émission. Le signal de tension comprend quatorze valeurs. Les moyens de traitement 18 dérivent le signal de tension en fonction de la fréquence d'émission pour obtenir un signal dérivé à treize valeurs.

Le signal dérivé est comparé avec les premières données de référence, et donc avec le signal dérivé de référence, en utilisant ici un test du $\chi^2$ à 5%.

Si le signal dérivé correspond bien à des doigts humains, le processeur 21 des moyens de traitement 18 ne détecte pas d'anomalie. Au contraire, si le signal dérivé s'étend au-delà des limites propres au corps humain, définies par la courbe de référence minimale et la courbe de référence maximale, les moyens de traitement 18 détectent qu'un doigt factice est utilisé.

On note que la comparaison du signal dérivé avec les premières données de référence, qui sont des valeurs pré-calculées et enregistrées, est très rapide et nécessite peu de mémoire, de sorte que cette comparaison est parfaitement adaptée à sa mise en oeuvre par le processeur d'une carte à puce.

Pour réaliser la détection, les moyens de traitement 18 peuvent aussi estimer un déphasage de chaque deuxième signal par rapport au premier signal associé, produire un signal de déphasage en fonction de la fréquence d'émission, et comparer le signal de déphasage avec les deuxièmes données de référence stockées dans la mémoire de la carte à puce 1. La comparaison peut alors être faite avec un test du $\chi^2$ à 5%.

Bien entendu, l'invention n'est pas limitée au mode de réalisation décrit mais englobe toute variante entrant dans le champ de l'invention telle que définie par les revendications.

Le circuit de détection est ici mis en oeuvre avec seulement deux électrodes (pour deux doigts), ce qui est très avantageux par rapport aux dispositifs connus d'analyse d'impédance des doigts humains, qui requièrent fréquemment quatre électrodes par doigt. Bien que cela ne soit pas nécessaire, il bien sûr possible ici aussi d'utiliser un nombre d'électrodes plus important.

Les électrodes peuvent se trouver sur une même face de la carte à puce.

L'architecture du circuit de détection pourrait être différente. Par exemple, le générateur pourrait être inclus directement dans les moyens de traitement.

Les premiers signaux ne comprennent pas nécessairement des signaux de fréquences différentes, mais pourraient être des signaux monofréquences. La fréquence des premiers signaux peut ainsi être constante, par exemple égale à 1kHz.

Les premiers signaux ne sont pas nécessairement des signaux sinusoïdaux. Les premiers signaux pourraient comprendre un simple signal carré périodique de fréquence constante, par exemple égale à 1kHz. La forme des deuxièmes signaux permet alors de distinguer un doigt humain d'un doigt factice. A nouveau, on peut réaliser une comparaison en utilisant un test du $\chi^2$ à 5%.

La détection se base ici sur une comparaison de signaux dérivés ou bien de déphasages, mais pourrait être basée sur une comparaison de caractéristiques différentes. On pourrait par exemple comparer des caractéristiques de *swell* dans les deuxièmes signaux, c'est-à-dire l'amplitude et la durée de pics de tension survenant dans les deuxièmes signaux.

## Revendications

**1.** Carte à puce (1) comportant un capteur d'empreintes digitales (2), **caractérisée en ce que** la carte à puce comporte en outre un circuit de détection (5) comprenant au moins une première électrode (6) et une deuxième électrode (7) isolées entre elles et formant un circuit ouvert (8) dans le circuit de détection, la première électrode et la deuxième électrode étant disposées pour que, lorsqu'un utilisateur applique un premier doigt sur le capteur d'empreintes digitales, le premier doigt soit en contact avec la première électrode et l'utilisateur puisse appliquer un deuxième doigt sur la deuxième électrode de manière à former une boucle refermant le circuit ouvert, le circuit de détection (5) comprenant de plus un circuit de filtrage (10) relié à la première électrode et à la deuxième électrode, un générateur (17) agencé pour générer des premiers signaux aux bornes du circuit de filtrage, et des moyens de traitement (18) agencés pour acquérir des deuxièmes signaux sur l'une des première électrode et deuxième électrode et pour détecter à partir des deuxièmes signaux si le premier doigt et le deuxième doigt comprennent un doigt factice.

**2.** Carte à puce selon la revendication 1, dans laquelle la première électrode (6) est un contour (4) du capteur d'empreintes digitales (2).

**3.** Carte à puce selon la revendication 1, dans laquelle la première électrode est formée sur une surface d'acquisition (3) du capteur d'empreintes digitales (2).

**4.** Carte à puce selon l'une des revendications précédentes, dans laquelle la première électrode (6) et la deuxième électrode (7) sont positionnées sur deux faces opposées de la carte à puce (1).

**5.** Carte à puce selon l'une des revendications précédentes, dans laquelle les premiers signaux comprennent des signaux sinusoïdaux ayant chacun une fréquence d'émission distincte, les moyens de traitement (18) étant agencés pour acquérir un deuxième signal pour chaque fréquence d'émission.

**6.** Carte à puce selon la revendication 5, dans laquelle les moyens de traitement (18) sont agencés pour déterminer une tension représentative de chaque deuxième signal, de manière à obtenir un signal de tension en fonction de la fréquence d'émission.

**7.** Carte à puce selon la revendication 6, dans laquelle les moyens de traitement sont agencés pour dériver le signal de tension en fonction de la fréquence d'émission pour obtenir un signal dérivé, et pour comparer le signal dérivé avec des premières données de référence stockées dans une mémoire de la carte à puce (1).

**8.** Carte à puce selon la revendication 7, dans laquelle les premières données de référence proviennent de courbes de référence (31, 32, 33) obtenues, préalablement ou pendant la fabrication de la carte à puce, par des mesures réalisées sur des doigts humains réels.

**9.** Carte à puce selon la revendication 8, dans laquelle les premières données de référence comprennent des coefficients de fonctions d'interpolation des courbes de référence.

**10.** Carte à puce selon la revendication 8, dans laquelle les premières données de référence comprennent des points remarquables des courbes de référence.

**11.** Carte à puce selon la revendication 5, dans laquelle les moyens de traitement (18) sont agencés pour estimer un déphasage de chaque deuxième signal, pour produire un signal de déphasage en fonction de la fréquence d'émission, et pour comparer le signal de déphasage avec des deuxièmes données de référence stockées dans une mémoire de la carte à puce (1).

**12.** Carte à puce selon la revendication 1, dans laquelle les premiers signaux sont des signaux monofréquences.

**Patentansprüche**

**1.** Chipkarte (1) mit Fingerabdrucksensor (2), **dadurch gekennzeichnet, dass** die Chipkarte außerdem einen Erkennungsschaltkreis (5) umfasst, der mindestens eine erste Elektrode (6) und eine zweite Elektrode (7) umfasst, die voneinander getrennt sind und einen offenen Schaltkreis (8) im Erkennungsschaltkreis bilden, wobei die erste Elektrode und die zweite Elektrode so angeordnet sind, dass, wenn ein Benutzer einen ersten Finger auf den Fingerabdrucksensor auflegt, der erste Finger in Kontakt mit der ersten Elektrode ist und der Benutzer einen zweiten Finger auf die zweite Elektrode auflegen kann, um eine Schleife zu bilden, die den offenen Schaltkreis schließt, wobei der Erkennungsschaltkreis (5) außerdem einen Filterschaltkreis (10), der mit der ersten Elektrode und der zweiten Elektrode verbunden ist, einen Generator (17), der dazu eingerichtet ist, erste Signale an den Anschlüssen des Filterschaltkreises zu erzeugen, und Verarbeitungsmittel (18) umfasst, die dazu eingerichtet sind, zweite Signale an einer von der ersten Elektrode und der zweiten Elektrode zu erfassen und um auf Grundlage der zweiten Signale zu erkennen, ob der erste Finger und der zweite Finger eine Fingerattrappe umfassen.

**2.** Chipkarte nach Anspruch 1, wobei die erste Elektrode (6) ein Umriss (4) des Fingerabdrucksensors (2) ist.

**3.** Chipkarte nach Anspruch 1, wobei die erste Elektrode auf einer Erfassungsfläche (3) des Fingerabdrucksensors (2) gebildet ist.

**4.** Chipkarte nach einem der vorhergehenden Ansprüche, wobei die erste Elektrode (6) und die zweite Elektrode (7) auf zwei entgegengesetzten Seiten der Chipkarte (1) positioniert sind.

**5.** Chipkarte nach einem der vorhergehenden Ansprüche, wobei die ersten Signale Sinussignale umfassen, die jeweils eine eigene Sendefrequenz haben, wobei die Verarbeitungsmittel (18) dazu eingerichtet sind, ein zweites Signal für jede Sendefrequenz zu erfassen.

**6.** Chipkarte nach Anspruch 5, wobei die Verarbeitungsmittel (18) dazu eingerichtet sind, eine für jedes zweite Signal repräsentative Spannung zu bestimmen, um so ein Spannungssignal in Abhängigkeit von der Sendefrequenz zu erhalten.

**7.** Chipkarte nach Anspruch 6, wobei die Verarbeitungsmittel dazu eingerichtet sind, das Spannungssignal in Abhängigkeit von der Sendefrequenz abzuleiten, um ein abgeleitetes Signal zu erhalten und um das abgeleitete Signal mit ersten Referenzdaten zu vergleichen, die in einem Speicher der Chipkarte (1) gespeichert sind.

**8.** Chipkarte nach Anspruch 7, wobei die ersten Referenzdaten von Referenzkurven (31, 32, 33) stammen, die zuvor oder während der Herstellung der Chipkarte durch Messungen an echten menschlichen Fingern erhalten wurden.

**9.** Chipkarte nach Anspruch 8, wobei die ersten Referenzdaten Koeffizienten von Funktionen zur Referenzkurveninterpolation umfassen.

**10.** Chipkarte nach Anspruch 8, wobei die ersten Referenzdaten bemerkenswerte Punkte der Referenzkurven umfassen.

**11.** Chipkarte nach Anspruch 5, wobei die Verarbeitungsmittel (18) dazu eingerichtet sind, eine Phasenverschiebung jedes zweiten Signals zu schätzen, um ein Phasenverschiebungssignal in Abhängigkeit von der Sendefrequenz zu erzeugen und um das Phasenverschiebungssignal mit zweiten Referenzdaten zu vergleichen, die in einem Speicher der Chipkarte (1) gespeichert sind.

**12.** Chipkarte nach Anspruch 1, wobei die ersten Signale Monofrequenzsignale sind.

**Claims**

**1.** Smart card (1) comprising a fingerprint sensor (2), **characterized in that** the smart card further comprises a detection circuit (5) comprising at least one first electrode (6) and one second electrode (7) that are isolated from one another and form an open circuit (8) in the detection circuit, the first electrode and the second electrode being disposed so that, when a user presses a first finger onto the fingerprint sensor, the first finger is in contact with the first electrode and the user can press a second finger onto the second electrode so as to form a loop closing the open circuit, the detection circuit (5) further comprising a filtering circuit (10) linked to the first electrode and to the second electrode, a generator (17) arranged to generate first signals at the terminals of the filtering circuit, and processing means (18) arranged to acquire second signals on one of the first electrode and second electrode and to detect from the second signals if the first finger and the second finger comprise a bogus finger.

2. Smart card according to Claim 1, wherein the first electrode (6) is an outline (4) of the fingerprint sensor (2).

3. Smart card according to Claim 1, wherein the first electrode is formed on an acquisition surface (3) of the fingerprint sensor (2).

4. Smart card according to one of the preceding claims, wherein the first electrode (6) and the second electrode (7) are positioned on two opposite faces of the smart card (1).

5. Smart card according to one of the preceding claims, wherein the first signals comprise sinusoidal signals each having a distinct emission frequency, the processing means (18) being arranged to acquire a second signal for each emission frequency.

6. Smart card according to Claim 5, wherein the processing means (18) are arranged to determine a voltage representative of each second signal, so as to obtain a voltage signal as a function of the emission frequency.

7. Smart card according to Claim 6, wherein the processing means are arranged to derive the voltage signal as a function of the emission frequency to obtain a derived signal, and to compare the derived signal with first reference data stored in a memory of the smart card (1).

8. Smart card according to Claim 7, wherein the first reference data originate from reference curves (31, 32, 33) obtained, prior to or during the manufacturing of the smart card, by measurements performed on real human fingers.

9. Smart card according to Claim 8, wherein the first reference data comprise coefficients of reference curve interpolation functions.

10. Smart card according to Claim 8, wherein the first reference data comprise noteworthy points of the reference curves.

11. Smart card according to Claim 5, wherein the processing means (18) are arranged to estimate a phase-shift of each second signal, to produce a phase-shift signal as a function of the emission frequency, and to compare the phase-shift signal with second reference data stored in a memory of the smart card (1).

12. Smart card according to Claim 1, wherein the first signals are single-frequency signals.

Figure 1

Figure 2

Figure 3

Figure 4

Figure. 5

Figure 6

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2006014205 A2